(19) Europäisches Patentamt
European Patent Office
Office européen des brevets

(11) EP 0 777 119 A2

(12) EUROPÄISCHE PATENTANMELDUNG

(43) Veröffentlichungstag:
04.06.1997 Patentblatt 1997/23

(51) Int. Cl.⁶: $G01N\ 21/47$, $A61B\ 5/00$

(21) Anmeldenummer: 96118421.5

(22) Anmeldetag: 16.11.1996

(84) Benannte Vertragsstaaten:
DE ES FR GB IT

(30) Priorität: 29.11.1995 DE 19544501

(71) Anmelder: BOEHRINGER MANNHEIM GMBH
68305 Mannheim (DE)

(72) Erfinder:
• Haar, Hans-Peter, Dr.
69168 Wiesloch (DE)

• Essenpreis, Matthias, Dr.
82131 Gauting (DE)
• Fritsche, Rainer, Dr.
68782 Brühl (DE)

(74) Vertreter: Pfeifer, Hans-Peter, Dr.,
Dr. H.-P. Pfeifer Dr. P. Jany,
Patentanwälte et al
Beiertheimer Allee 19
76137 Karlsruhe (DE)

(54) **Vorrichtung für Lichtreflexionsmessungen**

(57) Vorrichtung für Lichttransportmessungen an einem Meßobjekt (6) mit einem Meßkopf, der eine Kontaktfläche (3) zum Anlegen an eine Grenzfläche (5) des Meßobjektes aufweist, Lichteinstrahlungsmitteln (7) mit einem Lichtsender (20) zum Einstrahlen von Licht durch die Kontaktfläche (3) und eine Grenzfläche (5) in das Meßobjekt (6), und Detektionsmitteln (8) mit einem Lichtempfänger (21) zum Detektieren von aus dem Meßobjekt (6) austretendem Licht.

Die Kontaktfläche (3) weist mindestens eine optisch transparente Lichtpassagestelle (13,14) für das Licht auf, an der eine Vielzahl von starren Lichtleitelementen (15) angeordnet ist, wobei durch die Gesamtheit der Lichtleitelemente (15) einer Lichtpassagestelle (13,14) eine optische Verbindung zu einem der Lichtpassagestelle zugeordneten Lichtsender (20) oder Lichtempfänger (21) hergestellt wird.

**Fig. 1**

EP 0 777 119 A2

**Beschreibung**

Die Erfindung betrifft eine Vorrichtung für Lichttransportmessungen mit einem Meßkopf, der eine Kontaktfläche zum Anlegen an eine Grenzfläche des Meßobjektes aufweist.

Ein besonders wichtiges Anwendungsgebiet sind Lichttransportmessungen an Meßobjekten, die das Licht stark streuen, insbesondere an biologischem Gewebe, vor allem der Haut von Menschen oder Tieren. An der Haut werden Licht-Reflektionsmessungen vor allem bei medizinisch-analytischen Untersuchungen durchgeführt. Es sind eine Vielzahl von Verfahren vorgeschlagen worden, bei denen Licht unterschiedlicher Wellenlängen (vom UV bei etwa 200 nm bis ins Infrarot bei etwa 2.500 nm) verwendet wird. Soweit derartige Verfahren zur analytischen Bestimmung der Konzentration von in dem Gewebe enthaltenen Substanzen (Analyten) dienen, basieren sie vor allem auf den Prinzipien der Spektroskopie. Beispiele solcher Verfahren sind in der EP-0104772 A2 und den darin zitierten Druckschriften beschrieben.

Gemeinsam ist diesen Verfahren, daß von einem Lichtsender ausgehendes Licht ("Primärlicht") durch die Kontaktfläche des Meßkopfes und eine Grenzfläche des Meßobjektes (im Falle der Haut durch deren Oberfläche) in das Meßobjekt eingestrahlt und nach Wechselwirkung mit dem Meßobjekt aus diesem durch eine Grenzfläche austretendes Licht ("Sekundärlicht") detektiert wird. Dünne Meßobjekte (beispielsweise die Ohrläppchen) können von dem Licht durchstrahlt werden, d.h. die Detektion des Sekundärlichtes erfolgt an einer Grenzfläche, die der Einstrahlungs-Grenzfläche gegenüberliegt ("Transmissionsmessung"). Eine hierfür geeignete Lichttransport-Meßvorrichtung hat zwei Kontaktflächen, die an den beiden gegenüberliegenden Grenzflächen des Meßobjektes angelegt werden (vgl. z.B. US-2,706,927). Dabei kann die vorliegende Erfindung an einer oder an beiden der Kontaktflächen realisiert sein.

Besonders geeignet ist die Erfindung für Lichttransportmeßvorrichtungen, bei denen das Einstrahlen des Primärlichtes und die Detektion des Sekundärlichtes an der gleichen Grenzfläche erfolgt. Man spricht von einer Messung "in Reflexion", obwohl es nicht um die Reflexion im engeren Sinne an der Hautoberfläche geht, sondern auch hier das Licht in das Innere des Meßobjektes eingestrahlt und dort von dem Einstrahlungsort an einen Detektionsort transportiert wird, wobei der Lichttransport durch Absorption und Streuung in dem Meßobjekt bestimmt ist. Eine solche Vorrichtung kann man als Kontaktreflektometer bezeichnen.

In neuerer Zeit werden Kontaktreflektometer auch für Verfahren, die nicht nach spektroskopischen Prinzipien arbeiten, eingesetzt. Beispielsweise wird in der WO 94/10901 ein Verfahren und ein entsprechendes Kontaktreflektometer beschrieben, welches die Analyse von Glucose auf Basis der Streueigenschaften in dem Gewebe erlaubt.

Die Erfindung ist für diese und ähnliche Verfahren geeignet, jedoch nicht hierauf beschränkt. Sie ist generell überall dort erfolgreich einsetzbar, wo mit besonders hoher Genauigkeit Lichttransportmessungen in unmittelbarem Kontakt mit dem Meßobjekt durchgeführt werden müssen. Nichtbiologische Meßobjekte, für die die Erfindung geeignet ist, sind beispielsweise Teststreifenoberflächen, deren Farbe für eine bestimmte Analytkonzentration charakteristisch ist.

Um den Durchtritt des Lichtes zu ermöglichen, ist mindestens ein Teilbereich der Kontaktfläche optisch transparent. Ein solcher transparenter Teilbereich wird hier als Lichtpassagestelle bezeichnet. Im allgemeinen sind in der Kontaktfläche getrennte Lichtpassagestellen für die Einstrahlung des Primärlichtes und für die Detektion des Sekundärlichtes vorgesehen.

Insbesondere bei den genannten medizinisch-analytischen Anwendungen werden an ein geeignetes Meßgerät extrem hohe Anforderungen gestellt. Die Änderung des Sekundärlichtes in Abhängigkeit von der Konzentration des Analyten beträgt in dem gesamten medizinisch relevanten Konzentrationsbereich oft nur wenige Prozent. Um aus diesen geringen Änderungen die Analytkonzentration mit ausreichender Genauigkeit ableiten zu können, ist eine Meßgenauigkeit des Reflektometers in der Größenordnung von etwa 0,1% notwendig. Dabei kommt es vor allem auf die Stabilität und langfristige Reproduzierbarkeit der Messung an. Entscheidend ist also, daß ein bestimmter Lichtstrom des aus dem Meßobjekt austretenden Sekundärlichtes mit höchster Genauigkeit langfristig (mindestens über mehrere Stunden, nach Möglichkeit über mehrere Tage) zu dem gleichen elektrischen Signal führt. Vielfach sollen solche Geräte dem einzelnen Patienten für die laufende Überwachung eines kritischen Analyten (insbesondere der Glucose) zur Verfügung gestellt werden. Sie müssen deshalb trotz der hohen Anforderungen kostengünstig hergestellt werden können.

Der Erfindung liegt die Aufgabe zugrunde, bei einer Vorrichtung mit den vorstehend erläuterten Merkmalen eine Verbesserung der Meßgenauigkeit insbesondere hinsichtlich der Reproduzierbarkeit des Zusammenhanges zwischen dem Sekundärlichtstrom und dem gemessenen Signal zu erreichen.

Die Aufgabe wird bei einem solchen Meßgerät dadurch gelöst, daß die mindestens eine Lichtpassagestelle in der Kontaktfläche eine Vielzahl von starren Lichtleitelementen aufweist, wobei durch die Gesamtheit der Lichtleitelemente einer Lichtpassagestelle eine optische Verbindung zu einem der Lichtpassagestelle zugeordneten Lichtsender oder Lichtempfänger hergestellt wird.

Die optische Verbindung zwischen dem Meßobjekt und einem Lichtempfänger (Detektor) und/oder zwischen dem Meßobjekt und einem Lichtsender, wird also jeweils durch eine Lichtpassagestelle hergestellt, die dem jeweiligen Sender/Empfänger zugeordnet ist. Einer Lichtpassagestelle können mehrere Lichtsender zugeordnet sein, wie noch erläutert wird. Unter Umstän

den können einer Lichtpassagestelle auch mehrere Empfänger zugeordnet sein. Dies ist zum Beispiel sinnvoll, wenn ein Wellenlängenbereich zur Anwendung kommt, bei welchem zwei verschiedene Detektortypen für unterschiedliche Teilbereiche des Spektrums verwendet werden müssen ("Tandem-Detektor").

Wesentlich bei der Erfindung ist, daß das von einem bestimmten Lichtsender ausgehende Primärlicht und/oder das einem bestimmten Lichtempfänger (Detektor) zugeleitete Sekundärlicht an der Lichtpassagestelle der Kontaktfläche nicht durch eine unverschlossene Öffnung hindurchtritt und auch nicht durch ein einziges lichtleitendes Element wie beispielsweise ein Lichtleitstab transportiert wird, sondern durch eine große Anzahl von Lichtleitelementen. Vorzugsweise sind an einer Lichtpassagestelle - insbesondere in dem Fall, daß diese einem Lichtempfänger zugeordnet ist - mindestens 100, besonders bevorzugt mindestens 1000 Lichtleitelemente vorhanden sind. Weiterhin ist wichtig, daß die Lichtleitelemente starr sind, d.h. es soll sich nicht um biegbare Lichtleitfasern handeln, wie sie bisher weitgehend gebräuchlich waren.

Bei den genannten Verfahren haben die Lichtpassagestellen vielfach sehr kleine Abmessungen. Eine punktförmige Lichtpassagestelle von 0,5 mm Durchmesser kann im Falle der Erfindung gleichwohl über 1.000, möglicherweise sogar etwa 10.000 Lichtleitelemente aufweisen. Diese haben bevorzugt einen sehr kleinen Querschnitt von weniger als 0,01 mm$^2$, besonders bevorzugt weniger als 0,002 mm$^2$.

Geeignete Lichtleitelemente, die parallel zueinander dicht gepackt und damit trotz eines extrem kleinen Querschnittes starr sind, werden als sogenannte optische Faserplatten hergestellt.

Im Rahmen der Erfindung wurde festgestellt, daß bei den bisher gebräuchlichen Konstruktionen der lichtleitenden Elemente zwischen den optoelektronischen Wandlern (Lichtsendern und Lichtempfängern) und dem Meßobjekt vielfach bereits geringste mechanische Veränderungen im Sensorsystem oder an der Kontaktstelle zu der Grenzfläche des Meßobjektes (insbesondere der Hautoberfläche) Signaländerungen verursachen, die weit höher als die gewünschte Meßgenauigkeit von etwa 0,1% sind. Dadurch ist es oft nicht möglich, das gewünschte analytische Ergebnis mittel- und längerfristig mit ausreichender Genauigkeit zu bestimmen.

Im Rahmen der Erfindung wird wesentlich besser gewährleistet, daß ein immer gleichbleibender Bruchteil der von dem Sender ausgehenden Photonen tatsächlich in das Meßobjekt gelangt und - bei unverändertem Meßobjekt - ein ebenfalls gleichbleibender Bruchteil nach Verlassen des Meßobjektes durch die Grenzfläche zu dem Detektor gelangt. Die optische Stabilität ist insbesondere hinsichtlich Störeinflüssen durch kleine Unregelmäßigkeiten an der Oberfläche des Meßobjektes (Hautoberfläche) verbessert. Zugleich ist die Kontaktfläche geschlossen, so daß der Innenraum des Meßkopfes geschützt ist. Schließlich erlaubt die Erfindung, sowohl die Einstrahlung als auch die Detektion sehr präzise auf bestimmte Teilbereiche der Hautoberfläche ("Einstrahlungsort" und "Detektionsort" im Sinne der WO 94/10901) zu beschränken.

Die Erfindung wird nachfolgend anhand von in den Figuren dargestellten Ausführungsformen näher erläutert; es zeigen:

Fig. 1     Eine Prinzipdarstellung, teilweise im Schnitt, von einer erfindungsgemäßen Vorrichtung,

Fig. 2     eine Prinzip-Schnittdarstellung der wesentlichen Teile eines für die Erfindung geeigneten Meßkopfes,

Fig. 3     eine Aufsicht auf eine Detektoranordnung,

Fig. 4     eine stark vergrößerte und abstrahierte Detaildarstellung zur Erläuterung von für die Erfindung wesentlichen optischen Gesichtspunkten,

Fig. 5     eine Explosionsdarstellung der Optikeinheit einer Kontaktflächenbaugruppe für die Erfindung,

Fig. 6     eine perspektivische Darstellung eines Teils von alternativ verwendbaren Lichteinstrahlungsmitteln,

Fig. 7     eine Explosionsdarstellung einer Kontaktflächenbaugruppe unter Verwendung einer abgewandelten Optikeinheit,

Fig. 8     eine Ausschnittsdarstellung, teilweise im Schnitt und teilweise perspektivisch, von einer anderen Ausführungsform einer Kontaktflächenbaugruppe,

Fig. 9     eine perspektivische Darstellung der Kontaktflächenbaugruppe von Fig. 8, bei der die Halbleiterschicht senkrecht gestellt ist, um ihre Unterseite sichtbar zu machen.

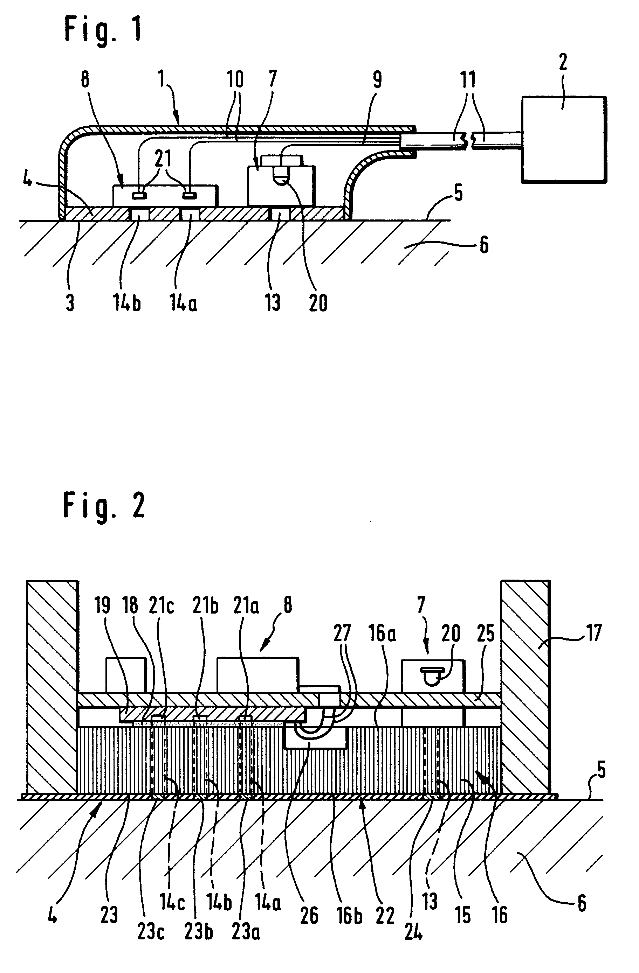

Das in Figur 1 stark schematisiert dargestellte Kontaktreflektometer besteht im wesentlichen aus einem Meßkopf 1 und einer Signalverarbeitungs- und Auswerteeinheit 2.

Der Meßkopf liegt mit der Kontaktfläche 3 einer Probenkontaktplatte 4 auf einer Grenzfläche 5 des zu untersuchenden Meßobjektes 6 auf. In dem Meßkopf 1 befinden sich insgesamt mit 7 bezeichnete Lichteinstrahlungsmittel und Detektionsmittel 8. Sie enthalten einen Lichtsender 20 (vorzugsweise einen Halbleiterlichtsender, insbesondere eine Leuchtdiode) und Lichtdetektoren 21 (vorzugsweise Halbleiterdetektoren, insbesondere Photodioden, Phototransistoren oder Avalanchephotodioden), die über elektrische Leitungen 9 bzw. 10 und ein Kabel 11 mit der Signalverarbeitungs- und Auswerteeinheit 2 verbunden sind.

Für den Durchtritt des Lichtes weist die Kontaktfläche 3 (und die Probenkontaktplatte 4 insgesamt) Lichtpassagestellen auf, wobei im dargestellten Fall eine Lichtpassagestelle 13 für das in das Meßobjekt 6 eingestrahlte Primärlicht und zwei Lichtpassagestellen 14a,14b zur Detektion von aus dem Meßobjekt 6 austretendem Sekundärlicht vorgesehen sind. Die Position und Größe der jeweiligen Primärlichtpassagestelle 13 (primary light passage location) und Sekundärlichtpassagestelle 14 (secondary light passage location) bestimmt den Ort der Grenzfläche, an dem das Licht in das Meßobjekt eingestrahlt ("Einstrahlungsort") bzw. aus diesem austretendes Licht detektiert ("Detektionsort") wird.

Die Signalverarbeitungs- und Auswerteeinheit 2 enthält die elektronischen Mittel, um die Lichteinstrahlungsmittel 7 anzusteuern und aus den von den Detektionsmitteln 8 erzeugten elektrischen Signalen (Meßsignalen) eine gewünschte Information über das Innere des Meßobjektes 6 abzuleiten. Wie einleitend bereits erläutert, ist die Erfindung für eine Vielzahl solcher Verfahren geeignet und die Signalverarbeitungs- und Auswerteeinheit 2 enthält die zu diesem Zweck jeweils erforderlichen, beispielsweise in den einleitend erwähnten Publikationen erläuterten Mittel. Hierzu gehören im Regelfall elektronische Verstärkerschaltungen (wie beispielsweise ein Lock-in-Verstärker) zur Aufbereitung des Meßsignals der Detektionsmittel sowie eine nachgeschaltete digitale Signalverarbeitungseinheit auf Basis eines Mikroprozessors.

Für die Erfindung wesentliche Besonderheiten sind in stark abstrahierter Form in Figur 2 dargestellt. Die Kontaktplatte 4 schließt dabei eine optische Faserplatte 16 ein, die aus einer Vielzahl von dicht gepackten, senkrecht zu der Kontaktfläche 3 verlaufenden, verhältnismäßig kurzen starren Lichtleitelementen 15 in Form von optischen Fasern besteht. Die Länge der Fasern und damit die Dicke der optischen Faserplatte 16 beträgt vorzugsweise höchsten 5 mm, besonders bevorzugt höchstens 2 mm. Eine Dicke von etwa 1 mm hat sich besonders bewährt.

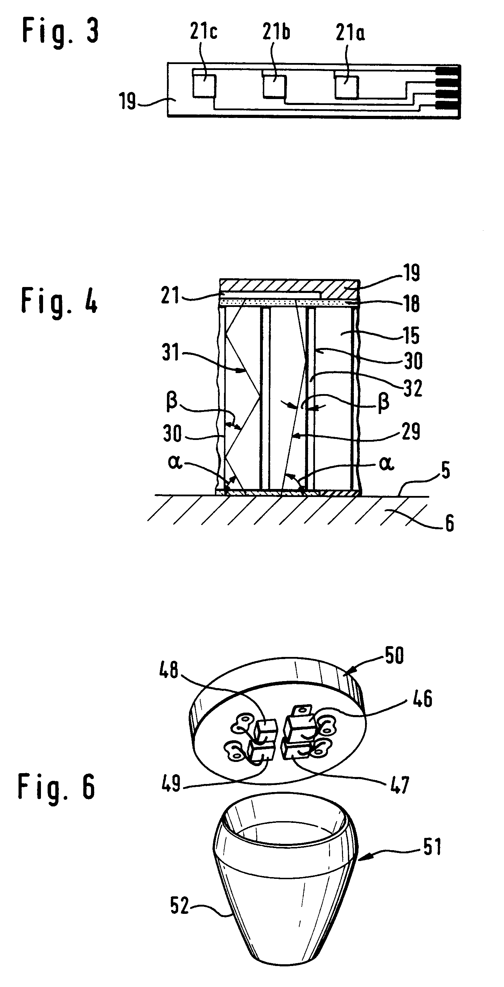

Bei der dargestellten Ausführungsform ist die optische Faserplatte 16 unmittelbar zwischen die Wände des Gehäuses 17 des Meßkopfes 1 derartig eingepaßt, daß sie das Gehäuse 17 zum Meßobjekt 6 hin vollständig abschließt. Sie ist mit Hilfe eines indexanpassenden Klebstoffes 18 direkt mit einer Halbleiterschicht 19 verklebt, die an entsprechenden Stellen lichtempfindliche Bereiche in Form von Siliziumdetektoren 21 (Photodioden) aufweist. Dies ist in Aufsicht in Figur 3 zu erkennen. Im dargestellten Fall sind drei Detektoren (Lichtempfänger) 21a, 21b und 21c vorgesehen. Mit diesen Detektoren fluchten Ausnehmungen in einer Maske 22, die wahlweise auf der detektorseitigen Oberfläche 16a oder der probenseitigen Oberfläche 16b der optischen Faserplatte 16 vorgesehen sein kann. Bei der dargestellten Ausführungsform ist auf der probenseitigen Oberfläche 16b eine Deckschicht 23 vorgesehen, die die Maske 22 bildet und an den Lichtpassagestellen 14a, 14b und 14c transparente Stellen 23a, 23b, 23c aus einer Antireflexbeschichtung aufweist, während die übrige Fläche aus schwarzem Lack besteht. Die Maske weist eine weitere transparente Stelle 24 auf, die die Lichtpassagestelle 13 für die Einstrahlung des Lichtes definiert.

Die Lichteinstrahlungsmittel 7 und die Detektionsmittel 8 werden durch eine zwischen den Wänden des Gehäuses 17 verlaufende Leiterplatte 25 gehalten. In der Faserplatte 16 ist eine Ausnehmung 26 vorgesehen, durch die Verbindungsdrähte 27 verlaufen, die die Verbindung zwischen den Detektorkontakten auf der Siliziumschicht 19 und den Leitern der Leiterplatte 25 herstellen.

Im Rahmen der Erfindung wurde festgestellt, daß eine mechanisch stabile Konstruktion der Optikeinheit wesentlich für die angestrebte hohe Meßgenauigkeit ist. Deswegen ist eine kompakte Bauweise besonders bevorzugt, bei der nachfolgende in Figur 2 dargestellte konstruktive Elemente einzeln oder in Kombination miteinander realisiert sind:

- Mindestens die Detektionsstellen, vorzugsweise aber auch die Einstrahlungsstellen sind in einer einzigen, gemeinsamen Faserplatte 16 vorgesehen.
- Die Maske 22 ist fest mit der Faserplatte verbunden. Als Maske und als meßobjektseitiger Abschluß der Faserplatte 16 eignet sich statt der vorstehend erwähnten Beschichtung auch eine selektiv schwarz eingefärbte Glasplatte, die durch Kitt oder durch ein Aufschmelzverfahren mit der optischen Faserplatte 16 verbunden ist.
- Die Detektoren sind mit der detektorseitigen Oberfläche 16a der Faserplatte fest und unbeweglich, insbesondere durch Kleben oder ein ähnliches dauerhaftes Fixierungsverfahren, verbunden.
- Die Detektoren 21 sind auf einem gemeinsamen Halbleitersubstrat 19 angeordnet. Hierdurch wird eine gleichartige Charakteristik der Detektionsempfindlichkeit erreicht. Außerdem ergibt sich durch Verkleben der Halbleiterplatte mit der Faserplatte eine hohe mechanische und optische Stabilität.

In Figur 4 sind drei Lichtleitelemente 15 in einer stark vergrößerten nicht maßstäblichen Darstellung zu erkennen. Dabei sind schematisch zwei Lichtstrahlen eingezeichnet, wobei der Lichtstrahl 29 den Weg von Photonen symbolisiert, die unter einem Winkel $\alpha$ von nahezu 90°, bezogen auf die Grenzfläche 5 austreten und deswegen in einem spitzen Winkel $\beta$ auf die Wände 30 der Lichtleitelemente 15 auftreffen, während der Lichtstrahl 31 unter einem spitzeren Winkel $\alpha$ aus der Grenzfläche 5 austritt, so daß der Auftreffwinkel $\beta$ der Photonen auf die Wand 30 hier größer ist. Bei der Erfindung sollen die optischen Bedingungen des Lichttransportes in den Lichtleitelementen 15 dergestalt sein, daß das Licht bis zu sehr kleinen Austrittswinkeln $\alpha$ (d.h. bis zu möglichst hohen Auftreffwinkeln $\beta$ der Lichtstrahlen auf die Wände 30) an den Wänden der Lichtleitelemente 15 vollständig reflektiert wird. Diese Eigenschaft wird als numerische Apertur NA bezeichnet: NA = n · sin $\beta$. Technisch ist die optische Apertur im Falle der erfindungsgemäß eingesetzten starren Lichtleitelemente durch die Reflexionseigenschaften an deren Wänden bestimmt, die wiederum vom Verhältnis der Brechungsindizes an der Wand und dem eventuellen Vorhandensein einer zusätzlichen Reflexionsschicht auf der Wand abhängen. Bevorzugt sollen die Lichtleitelemente eine numerische optische Apertur von mehr als 0,5 haben.

Die Photonen treten aus einem stark streuenden Meßobjekt 6 durch die Grenzfläche 5 isotrop (d.h. über einen weiten Winkelbereich gleichmäßig verteilt) aus. Die bei der Erfindung herrschenden optischen Verhältnisse führen dazu, daß alle diese Photonen vollständig oder zumindest mit einem langfristig stabilen konstanten Anteil zu dem jeweiligen Detektor 21 gelangen.

Die Lichtleitelemente einer Lichtpassagestelle leiten das Licht getrennt voneinander, sind optisch also voneinander weitgehend isoliert. Bei schlechter optischer Isolation wird die Meßgenauigkeit und Reproduzierbarkeit beeinträchtigt, wobei jedoch mit einem optischen Übersprechen (optical crosstalk) von weniger als 20% schon gute Ergebnisse erreicht werden, während andererseits selbst bei der extrem engen Anordnung starrer Lichtleitelemente in einer optischen Faserplatte problemlos ein optisches Übersprechen von weniger als 1% erreicht werden kann.

Die optische Isolation der Lichtleitelemente 15 ist in Figur 4 durch die Spalten 32 symbolisiert. In einer realen Faserplatte 16 sind die Lichtleitelemente 15 erheblich dichter gepackt, als dies in der Darstellung der Figur 4 erscheint, der Spalt 32 ist also wesentlich kleiner.

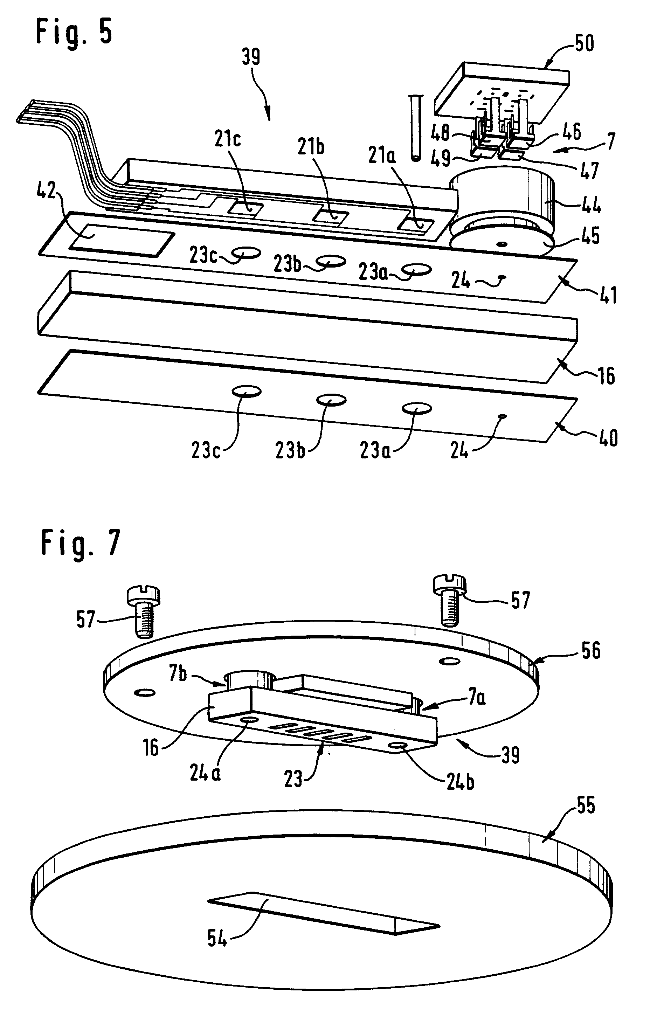

Bei der in Figur 5 dargestellten Optikeinheit 39 einer Kontaktflächenbaugruppe, die insbesondere für analytische Untersuchungen menschlicher Haut geeignet ist, ist eine optische Faserplatte 16 zwischen zwei Masken 40,41 sandwichartig eingeschlossen, wobei beide Masken 40,41 an den gleichen Stellen der Faserplatte 16 (also zueinander fluchtend) transparente Stelle 23a,23b,23c für Detektoren 21a,21b,21c sowie jeweils eine transparente Stelle 24 zum Einstrahlen von Licht aufweisen. Die Masken 40,41 können aus schwarzer Farbe, die im Siebdruckverfahren aufgebracht wird, bestehen. Besonders bevorzugt wird eine fotoempfindliche Schicht für die Maske verwendet, bei der die optischen Öffnungen durch einen Belichtungsprozeß (wie bei der Herstellung von Halbleiterplatten) erzeugt werden. Dieses Verfahren läßt sich besonders gut in den Herstellungsprozeß integrieren, ohne daß ein Vorrichtungswechsel nach dem Erzeugen der Maskenschicht erforderlich ist. Dadurch wird die präzise Montage erleichtert.

Die Verwendung von zwei Masken auf beiden Seiten der Faserplatte 16 ist vorteilhaft, weil die Maske auf der detektorseitigen Oberfläche 16a der Faserplatte 16 besonders präzise erzeugt und dieser Vorgang wie beschrieben in den Herstellungsprozeß integriert werden kann. Andererseits ist auch auf der probenseitigen Oberfläche 16b der Faserplatte 16 eine dunkle Maske - unabhängig von der präzisen Festlegung der Lichtpassagestellen - vielfach vorteilhaft, um Lichtanteile, die zwischen den Einstrahlungs- und Detektionsorten aus der Probenoberfläche austreten, zu absorbieren. Eine solche Maske kann mit etwas geringerer Präzision hergestellt und beispielsweise mit einem Druckverfahren aufgebracht sein.

Die detektorseitige Faserplatte 41 weist in Figur 5 eine zusätzliche Ausnehmung 42 zur Aufnahme der elektrischen Leiter auf, die die Verbindung zwischen den Detektoren 21a,21b,21c und der Meßelektronik herstellen.

Bei der in Figur 5 dargestellten Ausführungsform sind die Lichteinstrahlungsmittel 7 so gestaltet, daß an einer einzigen durch die transparente Stelle 24 der Masken 40,41 definierten Stelle des Meßobjektes Licht mehrerer unterschiedlicher Wellenlängen eingestrahlt werden kann. Zu diesem Zweck sind innerhalb eines Ulbricht-Zylinders 44, der nach unten (zu der Faserplatte 16 hin) durch eine innenseitig verspiegelte Schicht 45 verschlossen ist, vier Leuchtdioden 46 bis 48 angeordnet, die Licht mit unterschiedlichen Wellenlängen abstrahlen und an einer den Ulbricht-Zylinder nach oben abschließenden Deckplatte 50 befestigt sind.

Statt des Ulbricht-Zylinders 44 kann auch ein anderes Bauteil verwendet werden, welches bewirkt, daß das Licht der unterschiedlichen Leuchtdioden 46 bis 49 möglichst isotrop auf die gleiche Stelle auf der Oberfläche der Faserplatte 16 auftrifft. Ein solches optisches Element wird als "beam combiner" bezeichnet. Bevorzugt sollte ein für die Erfindung geeignetes beam-combiner-Element einen optischen Hohlraum aufweisen, dessen Wände (diffus oder spiegelnd) reflektieren, so daß das von Lichtsendern, die an unterschiedlichen Stellen an den Wänden des Hohlraumes angebracht sind, ausgehende Licht in dem Hohlraum isotrop verteilt wird.

Für eine ausreichend isotrope Verteilung sind die Dimensionen des Hohlraumes in Relation zu dem maximalen Lichtaustrittsabstand (Abstand zwischen den am weitesten voneinander entfernten Lichtaustrittspunkten) der Lichtsender von Bedeutung. Bevorzugt sollte der Abstand der Lichtsender von der Lichtaustrittsöffnung des beam-combiner-Elementes (d.h. der Lichteintrittsöffnung der zugeordneten Lichtpassagestelle) mindestens dreimal so groß und der mittlere Durchmesser des Hohlraumes mindestens zweimal so groß wie der maximale Lichtaustrittsabstand sein. Der optische Hohlraum des beam-combiner-Elementes muß nicht notwendigerweise leer sein. Geeignet ist beispielsweise ein kegelstumpfförmiges Teil aus einem transparenten Kunststoff, welches einen konischen Lichtleiter 51 bildet, der auf seiner Mantelfläche 52 versilbert ist, um eine Diffusionswirkung zu erreichen. Ein solcher beam combiner ist in Figur 6 dargestellt.

Die in Figur 5 dargestellte Ausführungsform mit einer Lichtpassagestelle für das Primärlicht und mehreren Lichtpassagestellen für das Sekundärlicht ermöglicht es, die Reflexionseigenschaften eines Meßobjektes für mehrere unterschiedliche Meßabstände zwischen dem jeweiligen Einstrahlungsort des Primärlichtes und dem jeweiligen Detektionsort des Sekundärlichtes zu bestimmen. Dies ist insbesondere bei Untersuchungen vorteilhaft, bei denen nicht nur das

optische Absorptionsverhalten (Absorptionskoeffizient $\mu_a$), sondern auch das Streuverhalten des Meßobjektes (Streukoeffizient $\mu_s$) untersucht werden soll. Solche Verfahren sind in den internationalen Patentanmeldungen WO 94/10901, WO 95/12348 und WO 95/32416 beschrieben. Dabei kann es alternativ auch vorteilhaft sein, mit mehreren Einstrahlungsorten und nur einem Detektionsort oder mit einer Kombination aus mehreren Einstrahlungs- und mehreren Detektionsstellen zu arbeiten.

Bei der in Figur 7 dargestellten Kontaktplattenbaugruppe weist die optische Einheit 39 beispielsweise zwei beam combiner als Teil der Lichteinstrahlungsmittel 7a,7b auf. Entsprechend sind an der Unterseite der Faserplatte 16 zwei transparente Stellen 24a,24b für das Primärlicht vorgesehen, welche zwei unterschiedliche Einstrahlungsorte auf der Hautoberfläche definieren. Weiterhin sind fünf transparente Stellen 23 für die Detektion entsprechend fünf unterschiedlichen Detektionsorten auf der Hautoberfläche vorhanden.

Figur 7 zeigt weiterhin eine zweckmäßige konstruktive Anordnung, bei der die Faserplatte 16 in eine entsprechende Ausnehmung 54 einer Hautkontaktplatte 55 eingelassen ist, die vorteilhafterweise aus Metall oder Glas bestehen kann. Eine solche Konstruktion, bei der die Hautkontaktplatte nur teilweise aus einer optischen Faserplatte besteht, ist aus Kostengründen vorteilhaft. Die Optikeinheit 39 ist beispielsweise wie dargestellt mit einer Halteplatte 56 und Schrauben 57 mit der Hautkontaktplatte 54 fest und stabil verbunden.

Bei der Ausführungsform gemäß Figur 7 sind zwei Lichtquellen (Lichteinstrahlungsmittel 7a und 7b) gemeinsam mit mehreren Detektoren an eine gemeinsame optische Faserplatte 16 angeschlossen. Die Erfindung erlaubt derartige Gestaltungen mit mehreren Einstrahlungsstellen und mehreren Detektionsstellen in besonders kompakter Bauweise und gleichzeitig hervorragender Meßgenauigkeit. Damit ist auch eine beispielsweise schachbrettartige Anordnung vieler Einstrahlungsstellen und Detektionsstellen mit vergleichsweise geringem konstruktivem Aufwand möglich.

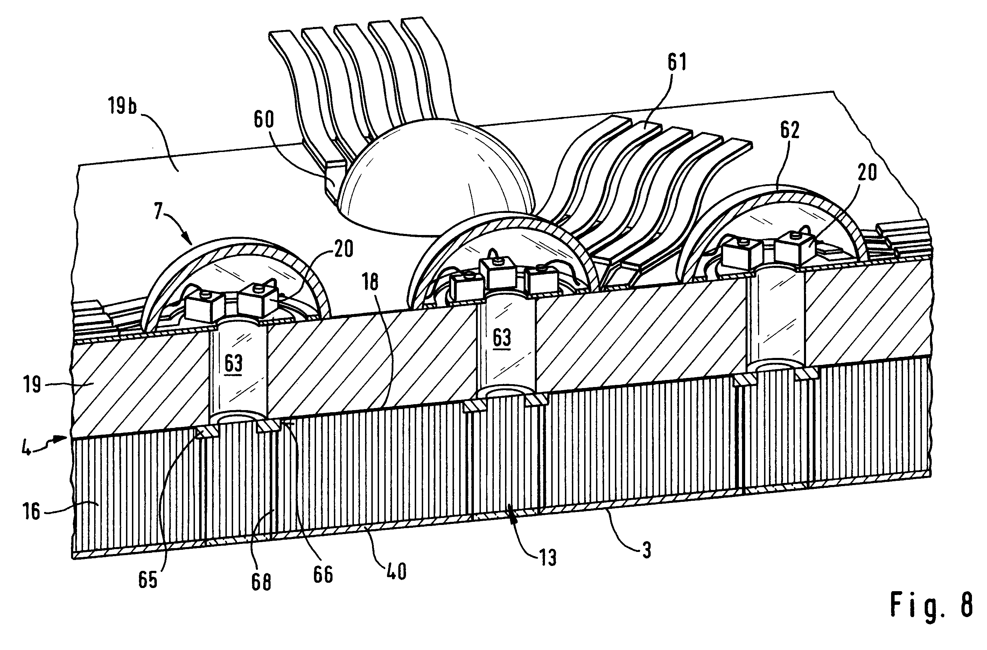

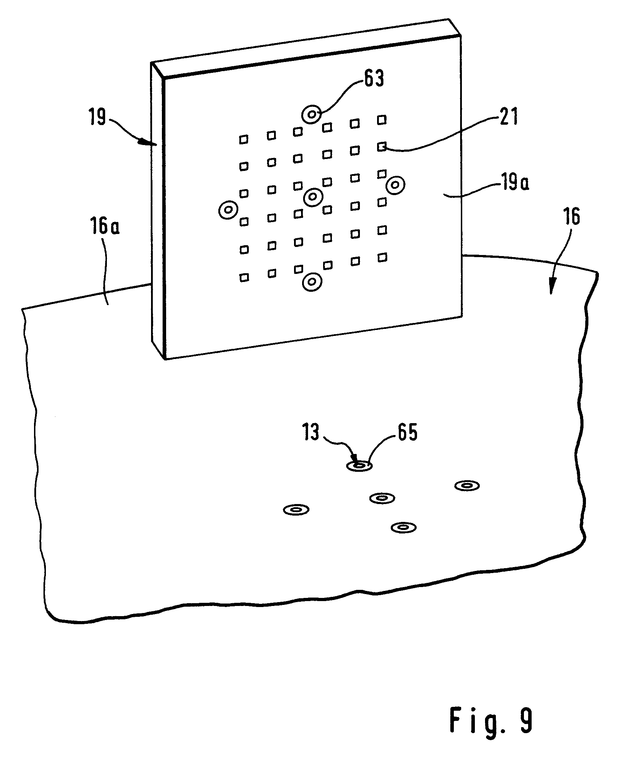

Eine solche Ausführungsform ist in den Fig. 8 und 9 dargestellt, wobei gleichzeitig einige weitere bevorzugte Ausführungsformen realisiert sind, die einzeln oder in Kombination miteinander bei der Erfindung verwendet werden können.

Fig. 8 zeigt einen Ausschnitt aus einer Probenkontaktplatte 4, die aus einer optischen Faserplatte 16 und einer Silizium-Halbleiterschicht 19 besteht, welche auf die Faserplatte 16 mit einer Schicht 18 eines indexangepaßten Klebstoffs aufgeklebt ist. Ebenso wie bei Fig. 2 sind die Detektoren 21 in die Halbleiterschicht 19 integriert, wobei auf der Unterseite 19a der Schicht schachbrettartig eine Vielzahl von Detektoren 21 (im dargestellten Fall 6 x 6 Detektoren mit einer Fläche von jeweils 0,25 x 0,25 mm) in einem Quadrat mit 10 mm Kantenlänge angeordnet sind.

Darüber hinaus sind bei dieser Ausführungsform auch die Lichtsender 20 an dem Halbleitersubstrat 19 befestigt, nämlich auf dessen Oberseite 19b aufgeklebt. Sie sind mit einem wirebond-Verfahren kontaktiert und über Dünnschicht-Leiterbahnen 60 und Kontaktfahnen 61 mit der Signalverarbeitungs- und Auswertungseinheit 2 verbunden. Ähnlich wie bei der Ausführungsform der Figuren 5 und 6 sind auch hier für jede Lichtpassagestelle 13 für das Primärlicht mehrere Lichtsender 20 in Form von Leuchtdioden unterschiedlicher Lichtwellenlängen vorgesehen. Sie strahlen das Licht im wesentlichen seitlich und nach oben ab. Ein beam-combiner-Element 62 bewirkt die isotrope Einstrahlung. Der optische Hohlraum des beam-combiners wird in diesem Fall von einer kuppelförmigen reflektierenden Abdeckung begrenzt.

An jeder Lichtpassagestelle 13 für das Primärlicht ist ein Lichtdurchtrittskanal 63 zwischen den Flächen der Siliziumschicht 19 vorgesehen, durch welchen das Primärlicht zu der Lichtpassagestelle 13 gelangt. Die Innenwand des Kanals 63 ist durch eine Metallisierung lichtreflektierend. Alternativ könnte auch ein in eine Bohrung der Siliziumschicht 19 eingelassener Lichtleitstab den Lichtdurchtrittskanal 63 bilden, wobei in diesem Fall die Bohrung innenseitig lichtabsorbierend eingefärbt ist.

Eine weitere Besonderheit der dargestellten Ausführungsform besteht darin, daß an jeder Lichtpassagestelle eine Oberflächen-Lichtbarriere 65 vorgesehen ist. Sie wird durch einen optischen Sperrgraben 66 gebildet. Dieser besteht aus einer in die Oberfläche der Faserplatte 16 (beispielsweise durch Ätzen) eingebrachten ringnutförmigen Vertiefung, die vorzugsweise mit einem optisch absorbierenden Material ausgefüllt ist. Dadurch wird ein optisches Übersprechen des Primärlichts an der Oberfläche der Faserplatte 16 wirksam verhindert. Wenn eine solche Oberflächen-Lichtbarriere 65 vorhanden ist, ist keine Maske auf der oberen Oberfläche der Faserplatte 16 notwendig, während auf der Haut-Seite der Faserplatte 16 vorzugweise eine Maske vorhanden sein sollte.

Ebenfalls zur Minimierung störender Reste von optischem Übersprechen in der Faserplatte 16 dient ein die Lichtpassagestelle 13 umgebender, vorzugsweise zylinderförmiger Mantel 68 aus einem absorbierenden Material. Beispielsweise können bei der Herstellung der Faserplatte 16 schwarz eingefärbte Glasfasern in Form einer die Lichtpassagestelle umgebenden Mantelfläche eingearbeitet werden.

**Patentansprüche**

1. Vorrichtung für Lichttransportmessungen an einem Meßobjekt (6) mit
   einem Meßkopf (1), der eine Kontaktfläche (3) zum Anlegen an eine Grenzfläche (5) des Meßobjektes aufweist,
   Lichteinstrahlungsmitteln (7) mit einem Lichtsender (20) zum Einstrahlen von Licht durch die Kontaktfläche (3) und die Grenzfläche (5) in das Meßobjekt (6), und

Detektionsmitteln (8) mit einem Lichtempfänger (21) zum Detektieren von durch die Grenzfläche (5) und die Kontaktfläche (3) aus dem Meßobjekt (6) austretendem Licht,
wobei die Kontaktfläche (3) mindestens eine optisch transparente Lichtpassagestelle (13,14) für das Licht aufweist, durch die eine optische Verbindung zu einem zugeordneten Lichtempfänger (21) oder Lichtsender (20) hergestellt wird.
**dadurch gekennzeichnet**, daß
die Lichtpassagestelle (13,14) in der Kontaktfläche eine Vielzahl von starren Lichtleitelementen (15) aufweist, durch die insgesamt die optische Verbindung zu dem zugeordneten Lichtsender (20) oder Lichtempfänger (21) hergestellt wird.

2. Vorrichtung nach Anspruch 1, **dadurch gekennzeichnet**, daß die Lichtpassagestelle (14) mindestens 100, bevorzugt mindestens 1000 Lichtleitelemente aufweist.

3. Vorrichtung nach Anspruch 1 oder 2, **dadurch gekennzeichnet**, daß die Lichtleitelemente (15) einen Querschnitt von weniger als 0,01 mm$^2$, bevorzugt weniger als 0,002 mm$^2$ haben.

4. Vorrichtung nach einem der vorhergehenden Ansprüche, **dadurch gekennzeichnet**, daß die Lichtleitelemente (15) eine numerische Apertur von mehr als 0,5 haben.

5. Vorrichtung nach einem der vorhergehenden Ansprüche, **dadurch gekennzeichnet**, daß in der Kontaktfläche (3) mehrere Lichtpassagestellen (13,14) vorgesehen sind, von denen mindestens eine einem Lichtempfänger (14) und eine andere einem Lichtsender (13) zugeordnet ist.

6. Vorrichtung nach einem der vorhergehenden Ansprüche, **dadurch gekennzeichnet**, daß die Länge der Lichtleitelemente (15) höchstens 5 mm, bevorzugt höchstens 2 mm beträgt.

7. Vorrichtung nach einem der vorhergehenden Ansprüche, **dadurch gekennzeichnet**, daß die Lichtleitelemente (15) parallel zueinander dicht gepackt angeordnete zu einer Platte (16) verbundene optische Faserstücke sind.

8. Vorrichtung nach Anspruch 7, **dadurch gekennzeichnet**, daß eine Mehrzahl von Lichtpassagestellen (13,14a bis 14c) in der Platte (16) vorgesehen und durch eine auf mindestens einer Seite der Platte (16) aufgebrachte Maske (22,40,41) voneinander getrennt sind.

9. Vorrichtung nach Anspruch 7 oder 8, **dadurch gekennzeichnet**, daß mindestens zwei in der Platte (16) vorhandene Lichtpassagestellen (13) durch eine Oberflächen-Lichtbarriere getrennt sind.

10. Vorrichtung nach einem der Ansprüche 7 bis 9, **dadurch gekennzeichnet**, daß mindestens eine in der Platte (16) vorhandene Lichtpassagestelle (13) von einem diese vollständig umgebenden Mantel (68) aus einem optisch absorbierenden Material umgeben ist.

11. Vorrichtung nach einem der Ansprüche 7 bis 10, **dadurch gekennzeichnet**, daß mindestens ein Lichtempfänger (21) mit der detektorseitigen Oberfläche (16a) der Platte (16) fest verbunden ist.

12. Vorrichtung nach Anspruch 11, **dadurch gekennzeichnet**, daß mehrere Lichtempfänger (21a bis 21c) auf dem gleichen Halbleitersubstrat angeordnet und mit der detektorseitigen Oberfläche (16b) der Platte (16) fest verbunden sind.

13. Vorrichtung nach Anspruch 12, **dadurch gekennzeichnet**, daß das Halbleitersubstrat mindestens einen durchgehenden Lichtdurchtrittskanal (63) aufweist, durch welchen Licht von einem Lichtsender (20) in das Meßobjekt eingestrahlt wird.

14. Vorrichtung nach einem der Ansprüche 7 bis 13, **dadurch gekennzeichnet**, daß mehrere Lichtsender (20), die Licht mit unterschiedlichen Wellenlängen abstrahlen, an einem Beam-Combiner-Element (44,51,62) derartig angeordnet sind, daß sie die gleiche Stelle der von der Kontaktfläche (3) abgewandten Seite der Platte (16) beleuchten.

15. Vorrichtung nach einem der vorhergehenden Ansprüche, für Lichttransportmessungen an biologischem Gewebe, insbesondere Hautgewebe, **dadurch gekennzeichnet**, daß sie eine Signalverarbeitungseinrichtung (2) einschließt, durch die das Meßsignal der Detektionsmittel zu einem Analyseergebnis weiterverarbeitet wird, das der Konzentration einer in der Haut enthaltenen Substanz entspricht.

Fig. 1

Fig. 2

Fig. 3

Fig. 4

Fig. 6

**Fig. 5**

**Fig. 7**

Fig. 8

Fig. 9